(19) Europäisches Patentamt
European Patent Office
Office européen des brevets

(11) Publication number: **0 058 173**
**B1**

(12) **EUROPEAN PATENT SPECIFICATION**

(45) Date of publication of patent specification: **05.12.84**

(51) Int. Cl.³: **C 07 C 41/01**, C 07 C 43/295, C 07 C 79/355, C 07 D 213/64, C 07 D 239/34, C 07 D 241/44

(21) Application number: **81902256.7**

(22) Date of filing: **06.08.81**

(86) International application number: **PCT/AU81/00104**

(87) International publication number: **WO 82/00638 04.03.82 Gazette 82/07**

# (54) PROCESS FOR THE SYNTHESIS OF ARYLOXY DERIVATIVES.

(30) Priority: **26.08.80 AU 5252/80**
**27.03.81 AU 8172/81**

(43) Date of publication of application:
**25.08.82 Bulletin 82/34**

(45) Publication of the grant of the patent:
**05.12.84 Bulletin 84/49**

(84) Designated Contracting States:
**CH DE FR GB LI**

(56) References cited:
**EP-A-0 017 767**
**WO-A-79/00094**
**US-A-3 966 826**

**E.E. Gilbert, "Sulphonation and Related Reactions", 1965, by Interscience Publishers, (New York), pages 379 to 381, *especially* page 380, lines 19-22.**

**The Merck Index, 9th Edition, 1976, (Merck & Co. Inc., N.J.), page ONR-27, "Elbs persulfate oxidation"**

**Tetrahedron, Volume 26, 1970 (Pergamon Press) pp. 5945-5951, Ogata et al, "Kinetics and Orientation in the Peroxydisulfate Oxidation of Phenol"**

(73) Proprietor: **ICI AUSTRALIA LIMITED**
**1 Nicholson Street**
**Melbourne Victoria 3001 (AU)**

(72) Inventor: **WATSON, Keith Geoffrey**
**36 Medway Street**
**Box Hill North,. Vic. 3129 (AU)**

(74) Representative: **Kneissl, Richard, Dr.**
**Widenmayerstrasse 46**
**D-8000 München 22 (DE)**

**EP 0 058 173 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

TECHNICAL FIELD

This invention relates to a process for the synthesis of organic compounds and in particular to a process for the synthesis of 4-(aryloxy)phenol derivatives.

BACKGROUND ART

4-(Aryloxy)phenol derivatives are useful intermediates for the synthesis of a wide range of [4-(aryloxy)phenoxy]alkane derivatives which have been shown to have herbicidal activity. In the past the required 4-(aryloxy)phenol derivatives have been prepared either:

i) by condensing the appropriate 4-alkoxyphenol with the appropriate aryl derivatives to give a [4-(alkoxy)phenoxy]aryl derivative and then cleaving the alkyl residue from the 4-alkoxy group; or

ii) by condensing the appropriate hydroquinone with the appropriate aryl derivative.

However, both of these processes suffer the disadvantage of using relatively expensive hydroquinone (derivatives) and the first process suffers the additional disadvantage of requiring the use of relatively expensive reagents to cleave the alkyl residue from the 4-alkoxy group.

DISCLOSURE OF INVENTION

It has now been found that 4-(aryloxy)phenol derivatives may be prepared from the appropriate phenol obviating the need to use a hydroquinone or a 4-alkoxyphenol.

Accordingly the invention provides a process for the synthesis of a compound of formula I

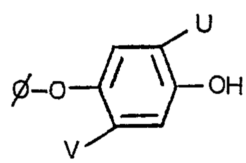

I

wherein:

Ø is chosen from aryl and heteroaryl groups of the formulae

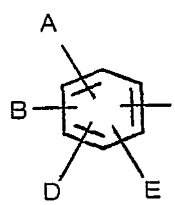
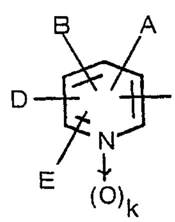
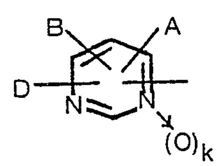

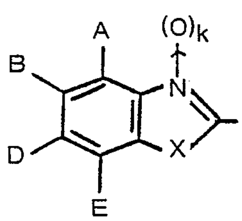
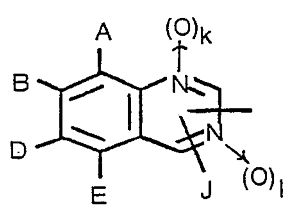
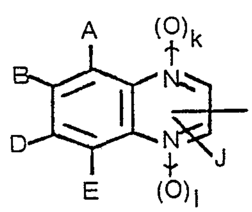

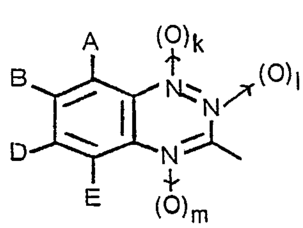
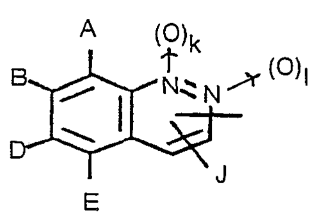

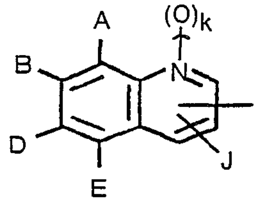

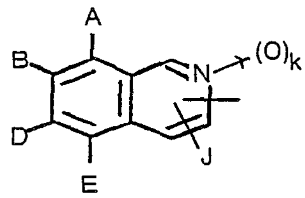

wherein A, B, D, E and J are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl)sulfamoyl, N,N-di($C_1$ to $C_6$ alkyl)sulfamoyl, carboxy, ($C_1$ to $C_6$ alkoxy)carbonyl, carbamoyl, N-($C_1$ to $C_6$ alkyl)carbamoyl, N,N-di($C_1$ to $C_6$ alkyl)carbamoyl, phenyl, phenoxy, phenylthio, and the groups substituted phenyl, substituted phenoxy and substituted phenylthio wherein in each group the phenyl ring is substituted with from 1 to 3 substituents chosen from the group consisting of halogen, $C_1$ to $C_6$ alkyl, $C_1$ to $C_6$ haloalkyl, $C_1$ to $C_6$ alkoxy, nitro and cyano; X is chosen from the group consisting of oxygen, sulfur and $NR^1$ wherein $R^1$ is chosen from hydrogen and $C_1$ to $C_6$ alkyl; k, l and m are independently chosen from 0 and 1 provided that k + l + m is 0, 1 or 2; and U and V are independently chosen from the group consisting of hydrogen, halogen, nitro, cyano, thiocyano, $C_1$ to $C_6$ alkyl, $C_1$ to $C_6$ haloalkyl, $C_2$ to $C_6$ alkenyl, $C_2$ to $C_6$ haloalkenyl, $C_1$ to $C_6$ alkoxy, $C_1$ to $C_6$ haloalkoxy, $C_1$ to $C_6$ alkylthio, carboxy, ($C_1$ to $C_6$ alkoxy)carbonyl, phenyl, phenoxy, phenylthio and the groups substituted phenyl, substituted phenoxy and substituted phenylthio wherein in each group the phenyl ring is substituted with from 1 to 3 substituents chosen from the group consisting of halogen, nitro, cyano, $C_1$ to $C_6$ alkyl, $C_1$ to $C_6$ haloalkyl and $C_1$ to $C_6$ alkoxy; which process is characterised in that it comprises the following steps in sequence:

a) reacting a sulfate ester of formula II,

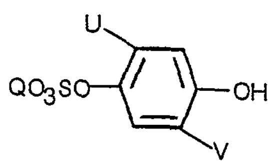

II

wherein Q is a cation chosen from hydrogen, the alkali and alkaline earth metals and ammonium, with a compound of formula III,

$$\phi\text{-L} \qquad \text{III}$$

wherein L is a leaving group chosen from chlorine, bromine, iodine, methanesulfonyl, trifluoromethanesulfonyl, *p*-toluenesulfonyl, methanesulfonyloxy, trifluoromethanesulfonyloxy, *p*-toluenesulfonyloxy, trifluoromethane sulfonamido and 1-pyrimidino *p*-toluenesulfonate; and

b) hydrolysing the sulfate ester formed in step a) to give a compound of formula I.

The sulfate ester of formula II may be readily formed by oxidation of a phenol with persulfuric acid or a salt thereof. Accordingly, in a preferred embodiment the invention provides a process for the synthesis of a compound of formula I as hereinbefore defined which process comprises:

i) oxidizing a compound of formula IV

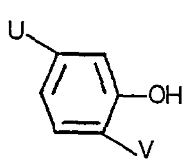

IV

with persulfuric acid or a salt thereof to form a sulfate ester of formula II

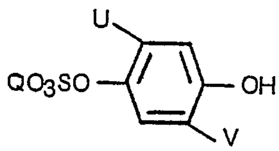

II

wherein U, V and Q are as defined above; and

ii) which process is characterised by the following steps in sequence:

a) reacting the sulfate ester of formula II with a compound of formula III,

Ø-L

III

wherein Ø and L are as defined above; and

b) hydrolysing the sulfate ester formed in step a) to give a compound of formula I.

Preferred A, B, D and E include hydrogen, halogen, nitro, cyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_1$ to $C_6$ alkoxy, $C_1$ to $C_6$ haloalkoxy, $C_1$ to $C_6$ alkylthio, carboxy and $C_1$ to $C_6$ alkoxycarbonyl. More preferred A, B, D and E include hydrogen, halogen, nitro and trifluoromethyl.

Preferred J include hydrogen, halogen, nitro, cyano, $C_1$ to $C_6$ alkyl and $C_1$ to $C_6$ haloalkyl. More preferred J include hydrogen and halogen.

Preferred X include oxygen, sulfur and $NR^1$ wherein $R^1$ is hydrogen or methyl. More preferred X include oxygen and sulfur.

Preferred k, l and m are 0 or 1 wherein k + l + m is 0 or 1. More preferred k, l and m are 0.

Preferred U and V include hydrogen, halogen, nitro, cyano, $C_1$ to $C_6$ alkyl, $C_1$ to $C_6$ haloalkyl, and $C_1$ to $C_6$ alkoxy. More preferred U and V include hydrogen and halogen.

In the compound of formula II preferred Q include the alkali metals such as, for example, sodium and potassium, and ammonium.

Step a) of the process of the invention may be carried out under a wide range of operating conditions. Preferably the reaction is carried out in the presence of an alkaline material.

Suitable alkaline materials include the alkali metal and alkaline earth metal hydroxides and carbonates such as, for example, sodium hydroxide, potassium hydroxide, sodium carbonate and potassium carbonate.

Step a) of the process of the invention is also preferably carried out in the presence of an organic solvent. Suitable solvents include: alcohols such as, for example, methanol, ethanol, n-propanol and isopropanol; ketones such as, for example, acetone, methyl ethyl ketone and methyl isobutyl ketone; and dipolar aprotic solvent such as, for example, dimethylformamide, dimethylacetamide, dimethylsulfoxide, N-methylpyrrolidone, hexamethylphosphoramide and sulfolan.

The specific reaction conditions required to effect the reaction in step a) of the process of the invention will vary with the specific reactants and solvent used. In general the reaction is facilitated by the application of heat and usually a reaction temperature in the range of from 40 to 150°C and a reaction time of between 0.5 and 20 hours is satisfactory. However, higher or lower reaction temperatures and/or shorter or longer reaction times may be employed if desired.

On completion of the reaction in step a) of the process of the invention the compound of formula I may be formed by hydrolysis of the sulfate ester according to step b) of the process of the invention. The sulfate ester formed in step a) of the process of the invention may be hydrolysed without isolation from the reaction mixture, or alternatively the sulfate ester may be isolated from the reaction mixture and then hydrolysed.

If step b) of the process of the invention is carried out without the isolation of the sulfate ester, conveniently, the compound of formula I may be formed in situ by acid hydrolysis. For example, the reaction mixture formed in step a) of the process of the invention may be acidified, for example with a mineral acid, for example, acetic acid, to hydrolyse the sulfate ester. In general the hydrolysis reaction is facilitated by the application of heat.

Alternatively, the sulfate ester formed in step a) of the process of the invention may be isolated from the reaction mixture before hydrolysis. Conveniently, the sulfate ester may be isolated by removing the solvent from the reaction mixture formed in step a) of the process of the invention. For example, if step a) of the process of the invention was carried out in aqueous solution the reaction

mixture may be extracted with a water-immiscible organic solvent, optionally after acidification of the reaction mixture, to remove any unreacted compound of formula III from the reaction mixture. The solvent may then be removed from the aqueous solution, for example by distillation, distillation under reduced pressure, evaporation or freeze-drying, and the sulfate ester may be purified as required. The sulfate ester may then be hydrolysed to form a compound of formula I, for example, by hydrolysis with an aqueous mineral acid such as hydrochloric acid or an organic acid such as acetic acid.

Preferably, the sulfate ester of formula II which is used in step a) of the process of the present invention is formed by the oxidation of a phenol of formula IV with persulfuric acid or a salt thereof. The oxidation may be carried out under a wide range of operating conditions. However, preferably the oxidation is carried out in aqueous alkaline solution using a salt of persulfuric acid. Suitable bases include the alkali metal and alkaline earth metal oxides, hydroxides and carbonates. Suitable salts of persulfuric acid include the ammonium salt and the alkali metal salts such as sodium persulfate and potassium persulfate. Conveniently, the compound of formula IV is dissolved in an aqueous alkaline solution and the oxidizing agent is added slowly to the solution which is maintained at or below ambient temperature during the addition.

On completion of the oxidation process the sulfate ester of formula II may be reacted directly, without isolation, with the compound of formula III or alternatively the sulfate ester may be isolated from the reaction mixture and then reacted with the compound of formula III.

If the step a) of the process of the invention is carried out without isolation of the sulfate ester, conveniently, the compound of formula III may be simply added to the oxidation reaction mixture and step a) of the process may be carried out in the reaction mixture formed in the oxidation process. If desired, any unreacted compound of formula IV may be removed from the oxidation reaction mixture before carrying out step a) of the process of the invention by, for example, acidifying the aqueous reaction mixture formed (e.g. by the addition of an acid or carbon dioxide) and extracting the aqueous acid solution with a relatively polar, water-immiscible organic solvent. Any unreacted compound of formula IV extracted into the organic solvent may be recovered and recycled.

Alternatively, the sulfate ester of formula II formed in the oxidation process may be isolated before reaction with the compound of formula III. Conveniently, the sulfate ester may be isolated by removing the solvent from the reaction mixture formed in the oxidation process and extracting the sulfate ester from the residue. For example, the aqueous reaction mixture formed may be acidified (e.g. by the addition of an acid or carbon dioxide) and extracted with a relatively polar, water-immiscible organic solvent to remove any unreacted compound of formula IV from the reaction mixture. The aqueous phase may then be made basic, for example made alkaline to litmus with sodium hydrogen carbonate. The solvent may then be removed from the alkaline solution, for example by distillation, distillation under reduced pressure, evaporation or freeze-drying, and the sulfate ester may then be extracted from the residue. The solvent used to extract the sulfate ester from the residue will depend to a large extent on the solubility properties of the sulfate ester. However, in general the solvent used will be a polar organic solvent, such as, for example, ethanol or acetone, in order to effect maximum recovery of the sulfate ester combined with as small an amount of inorganic salts as possible. After extraction of the sulfate ester, the sulfate ester containing solution may be used directly in step a) of the process of the invention or the sulfate ester may be separated for example by removal of the solvent, and further purified as required.

The process of the invention may be used for the preparation of a wide range of 4-(aryloxy)phenols of formula I. For example:

4-[(5-chloropyrimid-2-yl)oxy]phenol may be prepared by reacting a 4-hydroxyphenylsulfate salt with a 2-substituted-5-chloropyrimidine, such as 5-chloro-2-methanesulfonylpyrimidine, and hydrolysing the sulfate ester formed;

4-(2-nitro-4-trifluoromethylphenoxy)phenol may be prepared by reacting a 4-hydroxyphenylsulfate salt with a 4-substituted-3-nitrobenzotrifluoride, such as 4-chloro-3-nitrobenzotrifluoride, and hydrolysing the sulfate ester formed;

4-[(6-chloroquinoxalin-2-yl)oxy]phenol may be prepared by reacting a 4-hydroxyphenylsulfate salt with a 2-substituted-6-chloroquinoxaline, such as 2,6-dichloroquinoxaline, and hydrolysing the sulfate ester formed;

4-[(5-trifluoromethylpyrimidin-2-yl)oxy]phenol may be prepared by reacting a 4-hydroxyphenylsulfate salt with a 2-substituted-5-trifluoromethylpyrimidine, such as 2-chloro-5-trifluoromethylpyridine, and hydrolysing the sulfate ester formed;

4-(2,4-dichlorophenoxy)phenol may be prepared by reacting a 4-hydroxyphenylsulfate salt with a 2-substituted-2,4-dichlorobenzene such as 1-bromo-2,4-dichlorobenzene, and hydrolysing the sulfate ester formed; and

4-(4-trifluoromethylphenoxy)phenol may be prepared by reacting a 4-hydroxyphenylsulfate salt with a 4-substituted-benzotrifluoride, such as 4-chlorobenzotrifluoride, and hydrolysing the sulfate ester formed.

In the preferred process of the invention the 4-hydroxyphenylsulfate salt used in the foregoing reactions is prepared by the oxidation of phenol using a salt of persulfuric acid.

Modes of Carrying out the Invention

The invention is now illustrated by, but in no way limited to, the following Examples.

Example 1

Preparation of 4-[(5-chloropyrimid-2-yl)oxy]phenol

A solution of ammonium persulfate (8.0 g; 35 mmole) in water (30 ml) was added dropwise to a stirred solution of phenol (3.2 g; 35 mmole) in aqueous 10% sodium hydroxide (70 ml) which was maintained at or below a temperature of 20°C throughout the addition. On completion of the addition the solution was allowed to stand overnight at a temperature of 15 to 20°C. 5-Chloro-2-methanesulfonylpyrimidine (6.0 g; 30 mmole) was added and the reaction mixture was heated under reflux for a period of 1.5 hours. After cooling the reaction mixture was extracted with diethyl ether (2 × 100 ml). The aqueous phase was then made acidic to litmus by the addition of concentrated hydrochloric acid (10 ml). The reaction mixture was heated under reflux for a period of 1 hour. After cooling the reaction mixture was extracted with ethyl acetate (4 × 100 ml), the combined organic extracts were dried (over anhydrous magnesium sulfate), and the solvent was removed by distillation under reduced pressure. The residue was purified by chromatography over silica gel (eluent ethyl acetate) to give 4-[(5-chloropyrimid-2-yl)oxy]phenol (2.0 g).

The product was characterised by comparison with an authentic sample of 4-[(5-chloropyrimid-2-yl)oxy]phenol. The pmr spectrum of the product was identical with the pmr spectrum of the authentic sample and the chromatographic behaviour of the product on thin-layer chromatography was identical with that of the authentic sample.

Example 2

Preparation of 4-(2-nitro-4-trifluoromethylphenoxy)phenol

A solution of ammonium persulfate (8.0 g; 35 mmole) in water (30 ml) was added dropwise to a stirred solution of phenol (3.2 g; 35 mmole) in aqueous 10% sodium hydroxide (70 ml) which was maintained at or below a temperature of 20°C throughout the addition. On completion of the addition the solution was allowed to stand overnight at a temperature of 15 to 20°C. The solution was made acidic (to Congo red) by the addition of concentrated hydrochloric acid and the acidic solution was extracted with diethyl ether (2 × 500 ml). The aqueous phase was separated, made alkaline to litmus with aqueous sodium hydroxide solution and the water was removed by evaporation under reduced pressure. The residue was acidified and extracted with aqueous 90% ethanol (200 ml). The aqueous ethanolic solution was made alkaline and was heated under reflux while 4-chloro-3-nitrobenzotrifluoride (8.0 g; 35 mmole) was added dropwise. After heating under reflux for a period of 2 hours the hot solution was made acidic by the addition of concentrated hydrochloric acid and stirred overnight at room temperature. The solvent was removed by distillation under reduced pressure and the residue was partitioned between water and dichloromethane. The organic phase was separated, dried over anhydrous magnesium sulfate, and the solvent was removed by distillation under reduced pressure. The residue was chromatographed over silica gel (eluent dichloromethane) to give 4-(2-nitro-4-trifluoromethylphenoxy)phenol (0.6 g) as a pale yellow oil.

The product was characterised by comparison with an authentic sample of 4-(2-nitro-4-trifluoromethylphenoxy)phenol. The pmr spectrum of the product was identical with the pmr spectrum of the authentic sample and the chromatographic behaviour of the product on thin-layer chromatography was identical with that of the authentic sample.

Example 3

Preparation of 4-[(6-chloroquinoxalin-2-yl)oxy]phenol

a) Potassium 4-hydroxyphenylsulfate

A solution of potassium persulfate (27.0 g) in water (500 ml) was added dropwise over a period of 2 hours to a stirred solution of phenol (9.4 g) and sodium hydroxide (20.0 g) in water (200 ml) the reaction mixture being maintained at a temperature of 20±2°C throughout the addition. On completion of the addition the reaction mixture was stirred for a further period of 24 hours at a temperature of 20°C. An excess of carbon dioxide was passed through the reaction mixture to neutralize the base. The mixture was then extracted with diethyl ether (2 × 200 ml) and the unreacted phenol (2.7 g) was recovered from the ethereal extracts. The aqueous solution was evaporated to dryness under reduced pressure and the solid residue was extracted with an ethanol (20 parts)/water (1 part) mixture (3 × 500 ml). The combined aqueous ethanolic extracts were evaporated to dryness to give potassium 4-hydroxyphenylsulfate as a pale brown powder (10.5 g), mp 210—220°C. (Found: C, 31.7; H, 2.65; S, 13.9. $C_6H_5O_5SK$ requires: C, 31.6; H, 2.2; S, 14.0%).

b) 4-[(6-Chloroquinoxalin-2-yl)oxy]phenol

A mixture of potassium 4-hydroxyphenylsulfate (1.14 g; prepared as described in part a) above) 2,6-dichloroquinoxaline (0.90 g), anhydrous potassium carbonate (0.70 g), dimethylformamide (5 ml) and xylene (5 ml) was heated and stirred at a temperature of 110°C for a period of 5 hours. The solvents were removed by distillation under reduced pressure, acetic acid (20 ml) was added to the solid residue and the mixture was heated under reflux for a period of 2 hours. The acetic acid was

removed by distillation under reduced pressure and the solid residue was partitioned between water (100 ml) and ethyl acetate (2 × 200 ml). The combined organic extracts were dried over anhydrous magnesium sulfate and the solvent was removed by distillation under reduced pressure to give the crude product (1.8 g). The product was chromatographed over silica gel with chloroform elution to give recovered 2,6-dichloroquinoxaline (0.1 g), 2-[(6-chloroquinoxalin-2-yl)oxy]phenol (0.75 g; 64% based on 2,6-dichloroquinoxaline used) as a colourless crystalline solid, mp 204—205°C.

The product was characterised by comparison with an authentic sample of 4-[(6-chloroquinoxalin-2-yl)oxy]phenol. The pmr spectrum of the product was identical with the pmr spectrum of the authentic sample and the melting points and mixed melting point of the product and the authentic sample were the same.

Example 4

Preparation of 4-[(5-trifluoromethylpyridin-2-yl)oxy]phenol

A mixture of potassium 4-hydroxyphenylsulfate (1.14 g; prepared as described in Example 3 part a)), 2-chloro-5-trifluoromethylpyridine (0.93 g), anhydrous potassium carbonate (0.75 g), dimethylformamide (5 ml) and xylene (5 ml) was heated and stirred at a temperature of 85—90°C for a period of 4 hours. The solvents were removed by distillation under reduced pressure, acetic acid (15 ml) was added to the solid residue and the mixture was heated under reflux for a period of 2 hours. The acetic acid was removed by distillation under reduced pressure and the residue was partitioned between aqueous 1M sodium bicarbonate solution (100 ml) and chloroform (2 × 100 ml). The combined chloroform extracts were dried over anhydrous magnesium sulfate and the solvent was removed by distillation under reduced pressure to give the product as a pale brown oil. Chromatography over silica gel with chloroform elution gave 4-[(5-trifluoromethylpyridin-2-yl)oxy]phenol as an oil (0.35 g; 28%). Mass spectrum (m/e, %): 255 ($M^+$, 100); 254 (76); 227 (94).

Example 5

Preparation of 4-(2,4-dichlorophenoxy)phenol

A mixture of potassium 4-hydroxyphenylsulphate (2.0 g; prepared as described in Example 3 part a)), bromo-2,4-dichlorobenzene (2.0 g), anhydrous potassium carbonate (1.4 g), cuprous oxide (0.1 g) and pyridine (10 ml) was stirred and refluxed under an atmosphere of nitrogen for 16 hours. The pyridine was removed under reduced pressure, acetic acid (20 ml) was added to the residue and the mixture was heated under reflux for a period of 2 hours. The acetic acid was removed by distillation under reduced pressure and the residue was partitioned between 2 M aqueous sodium hydroxide (100 ml) and chloroform (50 ml). The aqueous layer was separated and acidified with hydrochloric acid and then extracted with chloroform (2 × 50 ml). The chloroform extracts were dried over anhydrous magnesium sulphate and the solvent was removed by distillation under reduced pressure to give the product as a brown oil (0.1 g). The 4-(2,4-dichlorophenoxy)phenol was identified by its pmr and mass spectra.

Proton magnetic resonance spectrum: ($CDCl_3$; $\delta$ in ppm) 6.7, d, 1H; 6.8, s, 4H; 7.1, d of d, 1H; 7.4, d, 1H.

Mass spectrum (m/e, %): 256 (70); 254 ($M^+$, 100); 220 (25), 184 (60).

Example 6

*4-(4-Trifluoromethylphenoxy)phenol* was prepared from 4-trifluoromethylchlorobenzene and potassium 4-hydroxyphenylsulfate following essentially the same procedure as that described in Example 5. The product a pale brown oil was characterized by its mass spectrum (m/e, %): 254 ($M^+$).

Industrial Applicability

The 4-(aryloxy)phenols which may be prepared according to the process of the invention are useful intermediates for the synthesis of a wide range of herbicidal, (aryloxyphenoxy)alkane derivatives. In particular, the 4-(aryloxy)phenol derivatives which may be prepared according to the process of the invention are useful intermediates in the preparation of a number of herbicidal 2-(4-aryloxyphenoxy)propionic acid derivatives.

The process of the present invention offers a number of advantages over the prior art processes which have been used for the synthesis of such 4-(aryloxy)phenol derivatives. In the past the required 4-(aryloxy)phenol derivatives have been prepared either:

i) by condensing the appropriate 4-alkoxyphenol with the appropriate aryl derivatives to give a [4-(alkoxy)phenoxy]aryl derivative and then cleaving the alkyl residue from the 4-alkoxy group; or

ii) by condensing the appropriate hydroquinone with the appropriate aryl derivative.

However, both of these processes suffer the disadvantage of using relatively expensive hydroquinone (derivatives), Moreover, the first process suffers the additional disadvantage of requiring the use of relatively expensive reagents to cleave the alkyl residue from the 4-alkoxy group while the second process suffers the additional disadvantage of possible reaction at both hydroxyl groups to give bis(aryloxy) derivatives of hydroquinones.

It will be evident to those skilled in the art that the process of the present invention offers the

advantage of the utilization of relatively inexpensive phenol (derivatives) and does not suffer the disadvantages of either the use of relatively expensive reagents or the possible formation of bis(aryloxy)derivatives of hydroquinones.

## Claims

1. A process for the synthesis of a compound of formula I

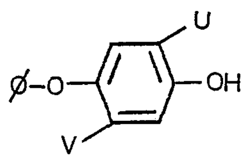

I

wherein:
Ø is chosen from aryl and heteroaryl groups of the formulae

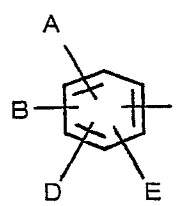

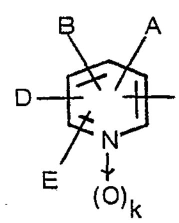

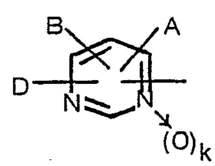

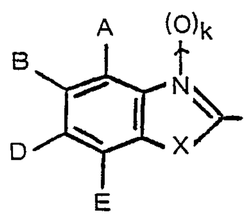

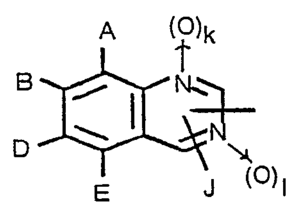

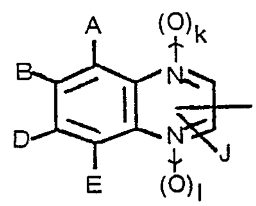

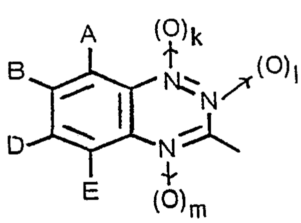

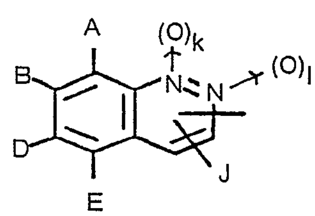

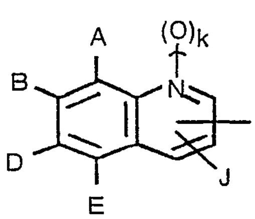

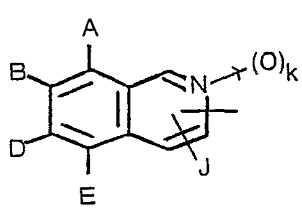

wherein A, B, D, E and J are independently chosen from the group consisting of hydrogen, halogen, nitro, cyano, thiocyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_3$ to $C_7$ cycloalkyl, $C_1$ to $C_6$ alkoxy, $C_1$ to $C_6$ haloalkoxy, $C_1$ to $C_6$ alkylthio, $C_1$ to $C_6$ alkylsulfinyl, $C_1$ to $C_6$ alkylsulfonyl, $C_1$ to $C_6$ haloalkylsulfinyl, $C_1$ to $C_6$ haloalkylsulfonyl, sulfo, $C_1$ to $C_6$ alkoxysulfonyl, sulfamoyl, N-($C_1$ to $C_6$ alkyl)sulfamoyl, N,N-di($C_1$ to $C_6$ alkyl)sulfamoyl, carboxy, ($C_1$ to $C_6$ alkoxy)carbonyl, carbamoyl, N-($C_1$ to $C_6$ alkyl)carbamoyl, N,N-di($C_1$ to $C_6$ alkyl)carbamoyl, phenyl, phenoxy, phenylthio, and the groups substituted phenyl, substituted phenoxy and substituted phenylthio wherein in each group the phenyl ring is substituted with from 1 to 3 substituents chosen from the group consisting of halogen, $C_1$ to $C_6$ alkyl, $C_1$ to $C_6$ haloalkyl, $C_1$ to $C_6$ alkoxy, nitro and cyano; X is chosen from the group consisting of oxygen, sulfur and $NR^1$ wherein $R^1$ is chosen from hydrogen and $C_1$ to $C_6$ alkyl;

k, l and m are independently chosen from 0 and 1 provided that k + l + m is 0, 1 or 2; and

U and V are independently chosen from the group consisting of hydrogen, halogen, nitro, cyano, thiocyano, $C_1$ to $C_6$ alkyl, $C_1$ to $C_6$ haloalkyl, $C_2$ to $C_6$ alkenyl, $C_2$ to $C_6$ haloalkenyl, $C_1$ to $C_6$ alkoxy, $C_1$ to $C_6$ haloalkoxy; $C_1$ to $C_6$ alkylthio, carboxy ($C_1$ to $C_6$ alkoxy)carbonyl, phenyl, phenoxy, phenylthio and the groups substituted phenyl, substituted phenoxy and substituted phenylthio wherein in each group the phenyl ring is substituted with from 1 to 3 substituents chosen from the group consisting of halogen, nitro, cyano, $C_1$ to $C_6$ alkyl, $C_1$ to $C_6$ haloalkyl and $C_1$ to $C_6$ alkoxy;

which process is characterised in that it comprises the following steps in sequence:

a) reacting a sulfate ester of formula II,

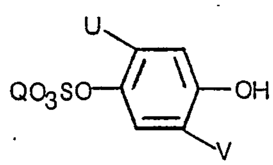

II

wherein Q is a cation chosen from hydrogen, the alkali and alkaline earth metals and ammonium, with a compound of formula III,

Ø-L

III

wherein L is a leaving group chosen from chlorine, bromine, iodine, methanesulfonyl, trifluoromethanesulfonyl, *p*-toluenesulfonyl, methanesulfonyloxy, trifluoromethanesulfonyloxy, *p*-toluenesulfonyloxy, trifluoromethane sulfonamido and 1-pyrimidino *p*-toluenesulfonate; and

b) hydrolysing the sulfate ester formed in step a) to give a compound of formula I.

2. A process for the synthesis of a compound of formula I as defined according to claim 1 which process comprises:

i) oxidizing a compound of formula IV

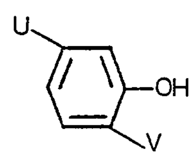

IV

with persulfuric acid or a salt thereof to form a sulfate ester of formula II

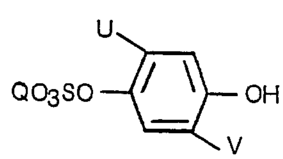

II

wherein U, V and Q are as defined according to claim 1; and

ii) which process is characterised by the following steps in sequence:
a) reacting the sulfate ester of formula II with a compound of formula III,

Ø-L III

wherein Ø and L are as defined according to claim 1; and

b) hydrolysing the sulfate ester formed in step a) to give a compound of formula I.

3. A process according to claim 1 wherein: step a) is carried out in the presence of a solvent and an alkaline material; and step b) is carried out using a mineral acid or an organic acid to hydrolyse the sulfate ester.

4. A process according to claim 2 wherein:

i) the oxidation of the compound of formula IV is carried out in aqueous alkaline solution using a salt of persulfuric acid as oxidant; and

ii) step a) is carried out in the presence of a solvent and an alkaline material; and step b) is carried out using a mineral acid or an organic acid to hydrolyse the sulfate ester.

5. A process according to claim 1 wherein: step a) is carried out in the presence of an organic solvent and an alkaline material chosen from the alkali metal and alkaline earth metal hydroxides and carbonates; and step b) is carried out using a mineral acid or an organic acid to hydrolyse the sulfate ester.

6. A process according to claim 2 wherein:

i) the oxidation of the compound of formula IV is carried out by the slow addition of a salt of persulfuric acid to an aqueous alkaline solution of the compound of formula IV; and

ii) step a) is carried out in the presence of an organic solvent and an alkaline material chosen from the alkali metal and alkaline earth metal hydroxides and carbonates; and step b) is carried out using a mineral acid or an organic acid to hydrolyse the sulfate ester.

7. A process according to claim 1 or claim 2 wherein:

in the compound of formula I — A, B, D and E are independently chosen from the group consisting of hydrogen, halogen, nitro, cyano, amino, $C_1$ to $C_6$ alkylamino, di($C_1$ to $C_6$ alkyl)amino, $C_1$ to $C_6$ alkyl, $C_1$ to $C_6$ haloalkyl, $C_2$ to $C_6$ alkenyl, $C_1$ to $C_6$ alkoxy, $C_1$ to $C_6$ haloalkoxy, $C_1$ to $C_6$ alkylthio, carboxy and $C_1$ to $C_6$ alkoxycarbonyl;

J is chosen from the group consisting of hydrogen, halogen, nitro, cyano, $C_1$ to $C_6$ alkyl and $C_1$ to $C_6$ haloalkyl;

X is chosen from the group consisting of oxygen, sulfur, and $NR^1$ wherein $R^1$ is hydrogen or methyl;

k, l and m are chosen from 0 and 1 wherein k + l + m is 0 or 1; and

U and V are independently chosen from the group consisting of hydrogen, halogen, nitro, cyano, $C_1$ to $C_6$ alkyl, $C_1$ to $C_6$ haloalkyl and $C_1$ to $C_6$ alkoxy.

8. A process according to claim 1 or claim 2 wherein:

in the compound of formula I — A, B, D and E are independently chosen from the group consisting of hydrogen, halogen, nitro and triufluoromethyl;

J is chosen from hydrogen and halogen;

X is chosen from oxygen and sulfur;

k, l and m are 0; and

U and V are independently chosen from hydrogen and halogen;

in the compound of formula II — Q is an alkali metal or ammonium; and

in the compound of formula III — L is chosen from the group consisting of chlorine bromine or iodine.

9. A process according to claim 1 or claim 2 wherein:

in the compound of formula I — Ø is chosen from

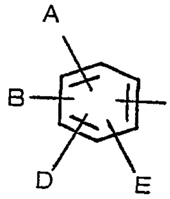
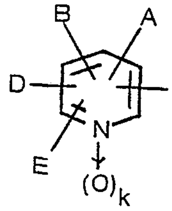
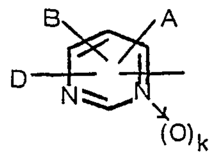
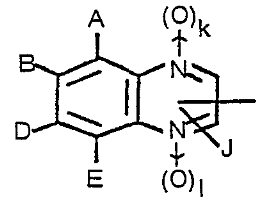

wherein A, B, D and E are independently chosen from the group consisting of hydrogen, halogen, nitro and trifluoromethyl;

J is hydrogen;

k, l and m are 0; and

U and V are hydrogen;

in the compound of formula II — Q is chosen from sodium, potassium and ammonium; and

in the compound of formula III — L is chosen from the group consisting of chlorine, bromine and iodine.

10. A process according to claim 1 or claim 2 for the synthesis of a compound of formula I selected from the group consisting of 4-[(5-chloropyrimid-2-yl)oxy]phenol, 4-(2-nitro-4-trifluoromethylphenoxy)phenol, 4-[(6-chloroquinoxalin-2-yl)oxy]phenol, 4-[(5-trifluoromethylpyridin-2-yl)oxy]phenol, 4-(2,4-dichlorophenoxy)phenol and 4-(4-trifluoromethylphenoxy)phenol.

**Patentansprüche**

1. Verfahren zur Synthese einer Verbindung der Formel I

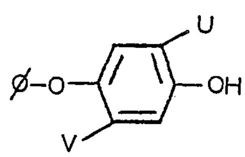

I

in der:

Ø aus Aryl- und Heteroaryl-Gruppen der Formeln ausgewählt ist:

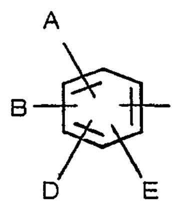

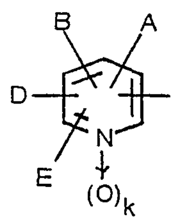

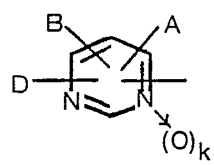

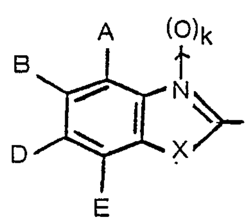

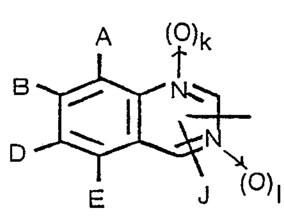

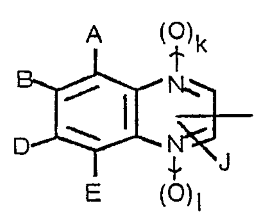

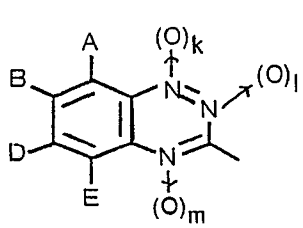

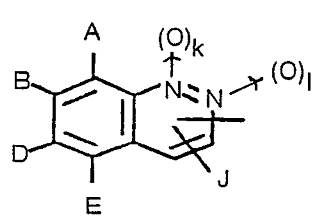

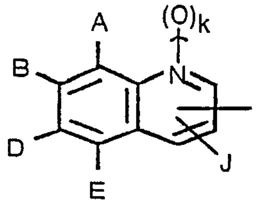

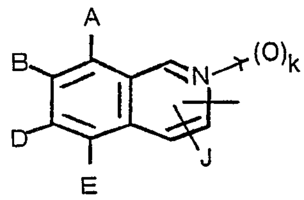

in denen A, B, D, E und J unabhängig voneinander aus einer Gruppe ausgewählt sind, die besteht aus Wasserstoff, Halogen, Nitro, Cyan, Thiocyan, Amino, $C_1$ bis $C_6$-Alkylamino, Di($C_1$ bis $C_6$-alkyl)amino, $C_1$ bis $C_6$-Alkyl, $C_1$ bis $C_6$ Halogenalkyl, $C_2$ bis $C_6$-Alkenyl, $C_3$ bis $C_7$-Cycloalkyl, $C_1$ bis $C_6$-Alkoxy, $C_1$ bis $C_6$-Halogenalkoxy, $C_1$ bis $C_6$-Alkylthio, $C_1$ bis $C_6$-Alkylsulfinyl, $C_1$ bis $C_6$-Alkylsulfonyl, $C_1$ bis $C_6$-Halogenalkylsulfinyl, $C_1$ bis $C_6$-Halogenalkylsulfonyl, Sulfo, $C_1$ bis $C_6$-Alkoxysulfonyl, Sulfamoyl, N-($C_1$ bis $C_6$-Alkyl)sulfamoyl, N,N-Di($C_1$ bis $C_6$-alkyl)sulfamoyl, Carboxy, ($C_1$ bis $C_6$-Alkoxy)carbonyl, Carbamoyl, N-($C_1$ bis $C_6$ Alkyl)carbamoyl, N,N-Di($C_1$ bis $C_6$-alkyl)carbamoyl, Phenyl, Phenoxy, Phenylthio sowie den Gruppen substituiertes Phenyl, substituiertes Phenoxy und substituiertes Phenylthio, wobei in jeder dieser Gruppen der Phenylring mit von 1 bis 3 Substituenten substituiert ist, die aus der Gruppe ausgewählt sind, die besteht aus Halogen, $C_1$ bis $C_6$-Alkyl, $C_1$ bis $C_6$-Halogenalkyl, $C_1$ bis $C_6$-Alkoxy, Nitro und Cyan;

X aus der Gruppe ausgewählt ist, die aus Sauerstoff, Schwefel und $NR^1$ besteht, worin $R^1$ aus Wasserstoff und $C_1$ bis $C_6$-Alkyl ausgewählt ist:

k, l und m unabhängig voneinander ausgewählt sind unter 0 und 1, vorausgesetzt, daß k + l + m 0, 1 oder 2 ist; und

U und V unabhängig voneinander aus der Gruppe ausgewählt sind, die besteht aus Wasserstoff, Halogen, Nitro, Cyan, Thiocyan, $C_1$ bis $C_6$-Alkyl, $C_1$ bis $C_6$-Halogenalkyl, $C_2$ bis $C_6$-Alkenyl, $C_2$ bis $C_6$-Halogenalkenyl, $C_1$ bis $C_6$-Alkoxy, $C_1$ bis $C_6$-Halogenalkoxy, $C_1$ bis $C_6$-Alkylthio, Carboxy, ($C_1$ bis $C_6$-Alkoxy)carbonyl, Phenyl, Phenoxy, Phenylthio sowie den Gruppen substituiertes Phenyl, substituiertes Phenoxy und substituiertes Phenylthio, wobei bei jeder dieser Gruppen der Phenylrest mit von 1 bis 3 Substituenten substituiert ist, die aus der Gruppe ausgewählt sind, die aus Halogen, Nitro, Cyan, $C_1$ bis $C_6$-Alkyl, $C_1$ bis $C_6$-Halogenalkyl und $C_1$ bis $C_6$-Alkoxy besteht;

wobei dieses Verfahren dadurch gekennzeichnet ist, daß es die folgenden aufeinanderfolgenden Schritte umfaßt:

a) Umsetzen eines Sulfatesters der Formel II

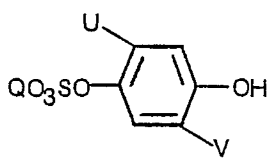

II

worin Q ein Kation ist, das aus Wasserstoff, den Alkali- und Erdalkali-Metallen und Ammonium ausgewählt ist,
mit einer Verbindung der Formel III

$$\text{Ø-L} \qquad \text{III}$$

worin L eine austretende Gruppe ist, die ausgewählt ist aus Chlor, Brom, Jod, Methansulfonyl, Trifluormethansulfonyl, p-Toluolsulfonyl, Methansulfonyloxy, Trifluormethansulfonyloxy, p-Toluolsulfonyloxy, Trifluormethan-sulfonamido und 1-Pyrimidino-p-toluolsulfonat; und

b) Hydrolysieren des in Stufe a) gebildeten Sulfatesters unter Bildung einer Verbindung der Formel I.

2. Verfahren für die Synthese einer Verbindung der Formel I, wie sie in Anspruch 1 definiert ist, wobei das Verfahren umfaßt:

i) Oxidieren einer Verbindung der Formel IV

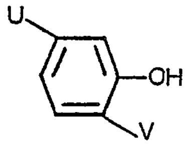

IV

mit Perschwefelsäure oder einem ihrer Salze unter Bildung eines Sulfatesters der Formel II

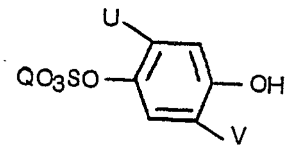

II

worin U, V und Q wie in Anspruch 1 definiert sind; und

ii) wobei das Verfahren durch die folgenden aufeinanderfolgenden Schritte gekennzeichnet ist:

a) Umsetzen des Sulfatesters der Formel II mit einer Verbindung der Formel III,

Ø-L III

worin Ø und L wie in Anspruch 1 definiert sind; und

b) Hydrolysieren des in Stufe a) gebildeten Sulfatesters unter Bildung einer Verbindung der Formel I.

3. Verfahren nach Anspruch 1, bei dem:

Stufe a) in Gegenwart eines Lösungsmittels und eines alkalischen Materials durchgeführt wird; und

Stufe b) unter Verwendung einer Mineralsäure oder einer organischen Säure zur Hydrolyse des Sulfatesters durchgeführt wird.

4. Verfahren nach Anspruch 2, bei dem:

i) die Oxidation der Verbindung der Formel IV in einer wässrigen alkalischen Lösung unter Verwendung eines Salzes der Perschwefelsäure als Oxidationsmittel durchgeführt wird; und

ii) die Stufe a) in Gegenwart eines Lösungsmittels und eines alkalischen Materials durchgeführt wird; und die Stufe b) unter Verwendung einer Mineralsäure oder einer organischen Säure zur Hydrolyse des Sulfatesters durchgeführt wird.

5. Verfahren nach Anspruch 1, bei dem:

Stufe a) in Gegenwart eines organischen Lösungsmittels und eines alkalischen Materials durchgeführt wird, das ausgewählt ist aus Alkalimetall- und Erdalkalimetall-hydroxiden und -carbonaten; und

Stufe b) unter Verwendung einer Mineralsäure oder einer organischen Säure zur Hydrolyse des Sulfatesters durchgeführt wird.

6. Verfahren nach Anspruch 2, bei dem:

i) die Oxidation der Verbindung der Formel IV durch langsame Zugabe eines Salzes der Perschwefelsäure zu einer wässrigen alkalischen Lösung der Verbindung der Formel IV durchgeführt wird; und

ii) Stufe a) in Gegenwart eines organischen Lösungsmittels und eines alkalischen Materials durchgeführt wird, das ausgewählt ist aus den Alkalimetall- und Erdalkalimetall-hydroxiden und -carbonaten; und

Stufe b) unter Verwendung einer Mineralsäure oder einer organischen Säure zur Hydrolyse des Sulfatesters durchgeführt wird.

7. Verfahren nach Anspruch 1 oder Anspruch 2, bei dem:

in der Verbindung der Formel I

A, B, D und E unabhängig voneinander aus der Gruppe ausgewählt sind, die besteht aus Wasserstoff, Halogen, Nitro, Cyan, Amino, $C_1$ bis $C_6$-Alkylamino, Di($C_1$ bis $C_6$-alkyl)amino, $C_1$ bis $C_6$-Alkyl, $C_1$ bis $C_6$-Halogenalkyl, $C_2$ bis $C_6$-Alkenyl, $C_1$ bis $C_6$-Alkoxy, $C_1$ bis $C_6$-Halogenalkoxy, $C_1$ bis $C_6$-Alkylthio, Carboxy und $C_1$ bis $C_6$-Alkoxycarbonyl;

J aus der Gruppe ausgewählt ist, die besteht aus Wasserstoff, Halogen, Nitro, Cyan, $C_1$ bis $C_6$-Alkyl und $C_1$ bis $C_6$-Halogenalkyl;

X aus der Gruppe ausgewählt ist, die besteht aus Sauerstoff, Schwefel und $NR^1$, worin $R^1$ Wasserstoff oder Methyl ist;

k, l und m ausgewählt sind aus 0 und 1, wobei k + l + m 0 oder 1 ist; und

U und V unabhängig voneinander aus der Gruppe ausgewählt sind, die besteht aus Wasserstoff, Halogen, Nitro, Cyan, $C_1$ bis $C_6$-Alkyl, $C_1$ bis $C_6$-Halogenalkyl und $C_1$ bis $C_6$-Alkoxy.

8. Verfahren nach Anspruch 1 oder Anspruch 2, bei dem:
in der Verbindung der Formel I

A, B, D, und E unabhängig voneinander aus der Gruppe ausgewählt sind, die aus Wasserstoff, Halogen, Nitro und Trifluormethyl besteht;

J ausgewählt ist aus Wasserstoff und Halogen;

X ausgewählt ist aus Sauerstoff und Schwefel;

k, l und m 0 sind; und

U und V unabhängig voneinander ausgewählt sind aus Wasserstoff und Halogen;

in der Verbindung der Formel II

Q ein Alkalimetall oder Ammonium ist; und

in der Verbindung der Formel III

L aus der Gruppe ausgewählt ist, die aus Chlor, Brom oder Jod besteht.

9. Verfahren nach Anspruch 1 oder Anspruch 2, bei dem:
in der Verbindung der Formel I

Ø ausgewählt ist aus

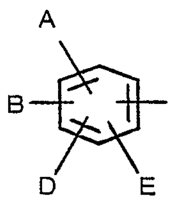

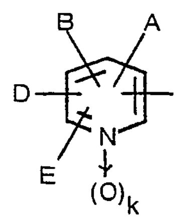

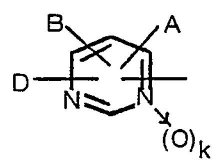

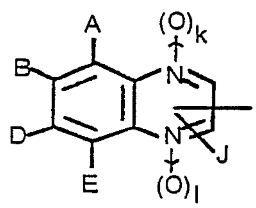

worin A, B, D und E unabhängig voneinander aus der Gruppe ausgewählt sind, die besteht aus Wasserstoff, Halogen, nitro und Trifluormethyl;

J Wasserstoff ist;

k, l und m 0 sind; und

U und V Wasserstoffe sind;

in der Verbindung der Formel II

Q ausgewählt ist aus Natrium, Kalium und Ammonium; und

in der Verbindung der Formel III

L ausgewählt ist aus der Gruppe, die aus Chlor, Brom und Jod besteht.

10. Verfahren nach Anspruch 1 oder Anspruch 2 zur Synthese einer Verbindung der Formel I, die ausgewählt ist aus der Gruppe, die besteht aus 4-[5-Chlorpyrimid-2-yl) oxy]-phenol, 4-(2-Nitro-4-trifluormethylphenoxy)phenol, 4-[(6-Chlorchinoxalin-2-yl)oxy]phenol, 4-[(5-Trifluormethylpyridin-2-yl)-oxy]phenol, 4-(2,4-Dichlorphenoxy)phenol und 4-(4-Trifluormethylphenoxy)phenol.

## Revendications

1. Procédé de synthèse d'un composé de formule I

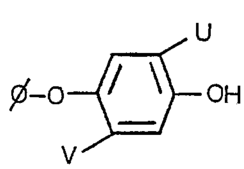

I

dans laquelle:

Ø est choisi entre des groupes aryle et des groupes hétéro-aryle de formules

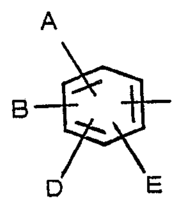
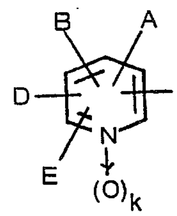
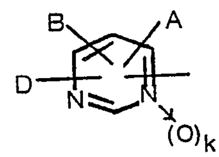

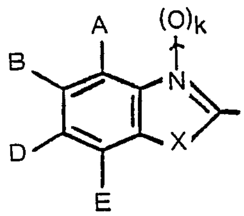
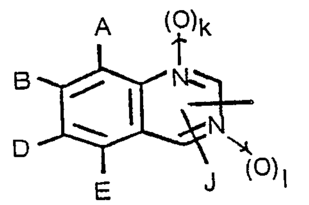
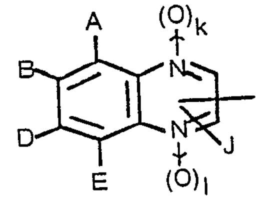

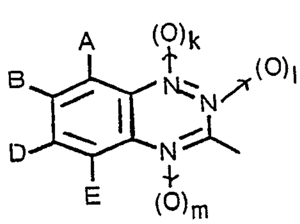
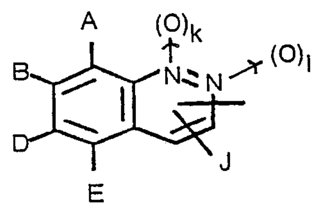

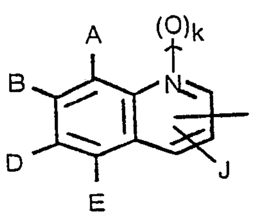
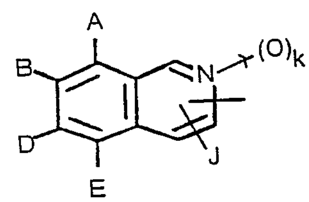

où A, B, D, E et J sont choisis, indépendamment, dans le groupe comprenant l'hydrogène, un halogène, un groupe nitro, cyano, thiocyano, amino, (alkyle en $C_1$ à $C_6$)amino, di-(alkyle en $C_1$ à $C_6$)amino, alkyle en $C_1$ à $C_6$, halogénalkyle en $C_1$ à $C_6$, alcényle en $C_2$ à $C_6$, cycloalkyle en $C_3$ à $C_7$, alkoxy en $C_1$ à $C_6$, halogénalkoxy en $C_1$ à $C_6$, alkylthio en $C_1$ à $C_6$, alkylsulfinyle en $C_1$ à $C_6$, alkylsulfonyle en $C_1$ à $C_6$, halogénalkylsulfinyle en $C_1$ à $C_6$, halogénalkylsulfonyle en $C_1$ à $C_6$, sulfo, alkoxysulfonyle en $C_1$ à $C_6$, sulfamoyle, N-(alkyle en $C_1$ à $C_6$)sulfamoyle, N,N-di-(alkyle en $C_1$ à $C_6$)sulfamoyle, carboxy, (alkoxy en $C_1$ à $C_6$)carbonyle, carbamoyle, N-(alkyle en $C_1$ à $C_6$)carbamoyle, N,N-di-(alkyle en $C_1$ à $C_6$)carbamoyle, phényle, phénoxy, phénylthio, et les groupes phényle substitués, phénoxy substitués et phénylthio substitués dans chacun desquels le noyau phényle est substitué avec 1 à 3 substituants choisis entre un halogène et des substituants alkyle en $C_1$ à $C_6$, halogénalkyle en $C_1$ à $C_6$, alkoxy en $C_1$ à $C_6$, nitro et cyano;

X est choisi dans le groupe comprenant l'oxygène, le soufre et $NR^1$ où $R^1$ est choisi entre l'hydrogène et un groupe alkyle en $C_1$ à $C_6$;

*k*, *l* et *m* sont choisis, indépendamment, entre 0 et 1, sous réserve que la somme k + l + m soit égale à 0, 1 ou 2; et

U et V sont choisis, indépendamment, dans le groupe comprenant l'hydrogène, un halogène, des groupes nitro, cyano, thiocyano, alkyle en $C_1$ à $C_6$, halogénalkyle en $C_1$ à $C_6$, alcényle en $C_2$ à $C_6$, halogénalcényle en $C_2$ à $C_6$, alkoxy en $C_1$ à $C_6$, halogénalkoxy en $C_1$ à $C_6$, alkylthio en $C_1$ à $C_6$, carboxy, (alkoxy en $C_1$ à $C_6$)carbonyle, phényle, phénoxy, phénylthio et les groupes phényle substitués, phénoxy substitués et phénylthio substitués dans chacun desquels le noyau phényle est substitué avec 1 à 3 substituants choisis dans le groupe comprenant un halogène, et des groupes nitro, cyano, alkyle en $C_1$ à $C_6$, halogénalkyle en $C_1$ à $C_6$ et alkoxy en $C_1$ à $C_6$;

procédé caractérisé en ce qu'il comprend les étapes successives ci-après:

a) réaction d'un ester sulfurique de formule II,

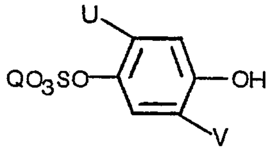

II

dans laquelle Q est un cation choisi entre l'hydrogène, les métaux alcalins et alcalino-terreux et l'ammonium, avec un composé de formule III,

Ø—L III

dans laquelle L est un groupe partant choisi entre chlore, brome, iode, méthanesulfonyle, trifluorométhanesulfonyle, *p*-toluènesulfonyle, méthanesulfonyloxy, trifluorométhanesulfonyloxy, *p*-toluènesulfonyloxy, trifluorométhanesulfamido et 1-pyrimidino-*p*-toluènesulfonate; et

b) hydrolyse de l'ester sulfurique formé dans l'étape a) pour obtenir un composé de formule I.

2. Procédé de synthèse d'un composé de formule I tel que défini dans la revendication 1, procédé qui consiste:

i) à oxyder un composé de formule IV

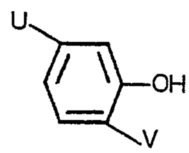

IV

avec l'acide persulfurique ou un sel de cet acide pour former un ester sulfurique de formule II

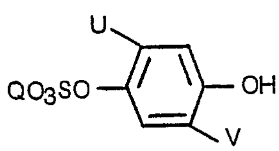

II

dans laquelle U, V et Q ont les définitions données dans la revendication 1; et

ii) procédé caractérisé par les étapes successives ci-après:

a) réaction de l'ester sulfurique de formule II avec un composé de formule III,

Ø—L III

ou Ø et L ont les définitions données dans la revendication 1; et

b) hydrolyse de l'ester sulfurique formé dans l'étape a) pour obtenir un composé de formule I.

3. Procédé suivant la revendication 1, dans lequel l'étape a) est conduite en présence d'un solvant et d'une matière alcaline; et l'étape b) est conduite en utilisant un acide minéral ou un acide organique pour hydrolyser l'ester sulfurique.

4. Procédé suivant la revendication 2, dans lequel:

i) l'oxydation du composé de formule IV est conduite en solution alcaline aqueuse en utilisant un sel d'acide persulfurique comme oxydant; et

ii) l'étape a) est conduite en présence d'un solvant et d'une matière alcaline; et l'étape b) est conduite en utilisant un acide minéral ou un acide organique pour hydrolyser l'ester sulfurique.

5. Procédé suivant la revendication 1, dans lequel:

l'étape a) est conduite en présence d'un solvant organique et d'une matière alcaline choisis entre

des hydroxydes et des carbonates de métaux alcalins et alcalinoterreux; et l'étape b) est conduite en utilisant un acide minéral ou un acide organique pour hydrolyser l'ester sulfurique.

6. Procédé suivant la revendication 2, dans lequel:

i) l'oxydation du composé de formule IV est conduite par lente addition d'un sel d'acide persulfurique à une solution alcaline aqueuse du composé de formule IV; et

ii) l'étape a) est conduite en présence d'un solvant organique et d'une matière alcaline choisie entre les hydroxydes et carbonates de métaux alcalins et alcalinoterreux; et l'étape b) est conduite en utilisant un acide minéral ou un acide organique pour hydrolyser l'ester sulfurique.

7. Procédé suivant la revendication 1 ou la revendication 2, dans lequel:

dans le composé de formule I —

A, B, D et E sont choisis, indépendamment, dans le groupe comprenant l'hydrogène, un halogène, des groupes nitro, cyano, amino, (alkyle en $C_1$ à $C_6$)amino, di-(alkyle en $C_1$ à $C_6$)amino, alkyle en $C_1$ à $C_6$, halogénalkyle en $C_1$ à $C_6$, alcényle en $C_2$ à $C_6$, alkoxy en $C_1$ à $C_6$, halogénalkoxy en $C_1$ à $C_6$, alkylthio en $C_1$ à $C_6$, carboxy et (alkoxy en $C_1$ à $C_6$)carbonyle;

J est choisi dans le groupe comprenant l'hydrogène, un halogène, les groupes nitro, cyano, alkyle en $C_1$ à $C_6$ et halogénalkyle en $C_1$ à $C_6$;

X est choisi dans le groupe comprenant l'oxygène, le soufre et $NR^1$, où $R^1$ est l'hydrogène ou le groupe méthyle;

*k*, *l* et *m* sont choisis entre 0 et 1, la somme $k + l + m$ étant égale à 0 ou à 1; et

U et V sont choisis, indépendamment, dans le groupe comprenant l'hydrogène, un halogène, des groupes nitro, cyano, alkyle en $C_1$ à $C_6$, halogénalkyle en $C_1$ à $C_6$ et alkoxy en $C_1$ à $C_6$.

8. Procédé suivant la revendication 1 ou la revendication 2, dans lequel:

dans le composé de formule I —

A, B, D et E sont choisis, indépendamment, dans le groupe comprenant l'hydrogène, un halogène, et les groupes nitro et trifluorométhyle;

J est choisi entre l'hydrogène et un halogène;

X est choisi entre l'oxygène et le soufre;

*k*, *l* et *m* sont égaux à 0; et

U et V sont choisis, indépendamment, entre l'hydrogène et un halogène;

dans le composé de formule II —

Q est un métal alcalin ou l'ion ammonium; et

dans le composé de formule III —

L est choisi entre le chlore, le brome et l'iode.

9. Procédé suivant la revendication 1 ou la revendication 2, dans lequel:

dans le composé de formule I —

Ø est choisi entre

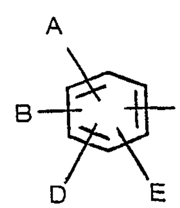

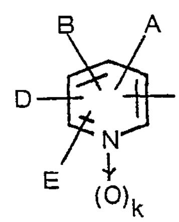

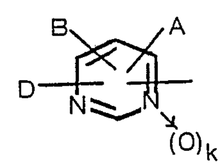

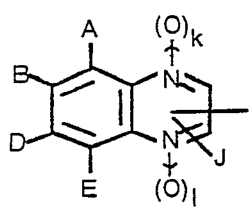

où A, B, D et E sont choisis, indépendamment, dans le groupe comprenant l'hydrogène, un halogène, le groupe nitro et le groupe trifluorométhyle;

J est l'hydrogène;

*k*, *l* et *m* sont égaux à 0; et

U et V représentent l'hydrogène;

dans le composé de formule II —

Q est choisi entre le sodium, le potassium et l'ion ammonium; et

dans le composé de formule III —

L est choisi dans le groupe comprenant le chlore, le brome et l'iode.

10. Procédé suivant la revendication 1 ou la revendication 2 pour la synthèse d'un composé de formule I choisi dans le groupe comprenant le 4-[(5-chloropyrimid-2-yl-oxy]-phénol, le 4-(2-nitro-trifluorométhylphénoxy)phénol, le 4-[(6-chloroquinoxaline-2-yl)oxy]-phénol, le 4-[(5-trifluorométhyl-pyridine-2-yl)oxy]-phénol, le 4-(2,4-dichlorophénoxy)phénol et le 4-(4-trifluorométhylphénoxy)phénol.